(19) Europäisches Patentamt
European Patent Office
Office européen des brevets

(11) EP 0 756 586 B1

(12) **EUROPÄISCHE PATENTSCHRIFT**

(45) Veröffentlichungstag und Bekanntmachung des Hinweises auf die Patenterteilung:
**29.10.1997 Patentblatt 1997/44**

(21) Anmeldenummer: 95918570.3

(22) Anmeldetag: **19.04.1995**

(51) Int. Cl.$^6$: **C04B 35/111**

(86) Internationale Anmeldenummer:
**PCT/EP95/01474**

(87) Internationale Veröffentlichungsnummer:
**WO 95/28364 (26.10.1995 Gazette 1995/46)**

(54) **AL2O3-SINTERMATERIAL, VERFAHREN ZU SEINER HERSTELLUNG UND VERWENDUNG DES MATERIALS**

AI2O3 SINTERING MATERIAL, PROCESS FOR THE PRODUCTION OF SAID MATERIAL AND USE THEREOF

PRODUIT FRITTE D'AL2O3, SON PROCEDE DE PRODUCTION ET SON UTILISATION

(84) Benannte Vertragsstaaten:
**AT CH DE ES FR GB IT LI NL SE**

(30) Priorität: **19.04.1994 EP 94106040**

(43) Veröffentlichungstag der Anmeldung:
**05.02.1997 Patentblatt 1997/06**

(73) Patentinhaber: **FRAUNHOFER-GESELLSCHAFT ZUR FÖRDERUNG DER ANGEWANDTEN FORSCHUNG E.V.**
**80636 München (DE)**

(72) Erfinder:
• **KRELL, Andreas**
  **D-01217 Dresden (DE)**
• **BLANK, Paul**
  **D-01187 Dresden (DE)**

(74) Vertreter: **Glawe, Delfs, Moll & Partner**
**Patentanwälte**
**Liebherrstrasse 20**
**80538 München (DE)**

(56) Entgegenhaltungen:
**EP-A- 0 406 847     EP-A- 0 408 771**
**EP-A- 0 519 159**

• **R.J.BROOK (ED.) 'CONCISE ENCYCLOPEDIA OF ADVANCED CERAMIC MATERIALS', PERGAMMON PRESS, OXFORD, UK, 203750 siehe Seite 304 - Seite 305; Tabellen 1-4**
• **JOURNAL OF THE AMERICAN CERAMIC SOCIETY, Bd. 71, Nr. 2, - 29.Februar 1988 WESTERVILLE,OHIO, USA, Seiten C74-C77, T.UEYAMA ET AL. 'PULVERIZATION AND DISPERSION TECHNIQUE FOR AGGLOMERATED PARTICLES OF ALUMINA POWDER IN A SLURRY'**
• **JOURNAL OF THE AMERICAN CERAMIC SOCIETY, Bd. 71, Nr. 10, - 31.Oktober 1988 WESTERVILLE, OHIO,USA, Seiten 841-844, XP 000081167 M.D.SACKS ET AL. 'LOW TEMPERATURE SINTERING OF AL2O3'**

**Beschreibung**

Die Erfindung bezieht sich auf das Gebiet der Keramik und betrifft $Al_2O_3$-Sintermaterialien und Verfahren zu ihrer Herstellung, die z.B. als Humanimplantat, als Verschleißprodukt, als Schneidwerkzeug oder als Schleifmittel zur Anwendung kommen können.

Die intensiven Bemühungen der letzten Jahre zur Herstellung monolithischer $Al_2O_3$-Sinterprodukte aus Korundpulver mit Gefügekorngrößen unter 2 µm und verbesserten mechanischen Eigenschaften erfolgten im wesentlichen auf zwei Wegen:

- Einsatz von möglichst feinkörnigen Submikrometer- bzw. Nanometer-Pulvern,
- Zusatz von Substanzen zur Senkung der Dichtsintertemperatur.

Die sehr feinkörnigen Ausgangssubstanzen haben zwar die erwünschte hohe Sinteraktivität, bringen aber wegen ihres schlechten Verdichtungsverhaltens gleichzeitig erhebliche Probleme bei der Formgebung mit sich. Das bisher überwiegend angewandte kostengünstige uniaxiale Trockenpressen führt zu unzureichender Gründichte bzw. zu Dichteinhomogenitäten im Formkörper und bei der Sinterung zu härte- und festigkeitsmindernden Defekten. Aus den genannten Gründen werden andere Formgebungsverfahren, wie kaltisostatisches Pressen, Strangziehen, Druck- bzw. Zentrifugalschlickerguß oder Gelcasting angewandt.

Mittels Strangpressens von keramischen Massen aus Submikrometerpulvern und Sinterung bei 1400 °C wurde eine $Al_2O_3$-Gefügekorngröße von 0,8 µm und eine Härte HV20 = 1920 erzielt (G. Riedel, u.a., Silicates Industriels (1989) 1/2,29-35). Das dabei eingesetzte Pulver hatte eine mittlere Korngröße von 0,45 µm und einen die Breite des Grobkornanteils der Korngrößenverteilung beschreibenden Wert $d_{84}$ = 1,0 µm.

Auch bei sehr feinkörnigen $Al_2O_3$-Pulvern mit $d_{50}$ < 0,4 µm und einem die Breite des Grobkornanteils der Korngrößenverteilung beschreibenden Wert $d_{84}$ < 0,7 µm wird neuerdings erfolgreich der Schlickerguß eingesetzt (T.-Sh. Yeh u.a., J. Am. Ceram. Soc. (1988),S.841-844), auch in Kombination mit der Anwendung von Druck (Druckfiltration: F.F. Lange u.a., Bull. Am. Ceram. Soc. (1987),S.1498-1504; Vakuum-Druckfiltration: H. Mizuta u.a., J. Am. Ceram. Soc. (1992),S.469-473). Wie z.B. von Mizuta u.a. gezeigt, können mittels dieser aufwendigen Verfahren die bisher besten mechanischen Kennwerte an reinem Sinterkorund erreicht werden, jedoch wurden nach diesem Stand der Technik selbst mittels heißisostatischen Pressens (HIP) keine Vickers-Kleinlasthärten $\geq$ 2000 oder Biegebruchfestigkeiten $\geq$ 800 MPa gemessen.

Die Nutzbarkeit druckloser Gießprozesse wie des sogenannten Gelcasting oder enzymgesteuerter Koagulation zur Herstellung von reiner $Al_2O_3$-Keramik war bisher auf Korundpulver mit mittleren Korngrößen von mehr als 0,4 µm beschränkt (A.C. Young, u.a., J. Am. Ceram. Soc. (1991)3, 612-618), so daß die mittleren Korngrößen der erzeugten dichten Sintergefüge stets größer als 1,5 µm waren. Die erreichten Festigkeiten blieben im zitierten Beispiel unter 300 MPa.

Die Möglichkeiten zur Verbesserung der mechanischen Eigenschaften und der Feinkörnigkeit von $Al_2O_3$-Sinterprodukten hergestellt aus Korundpulver durch Zusatz von die Sinterung fördernden Substanzen sind sehr beschränkt. Die Dichtsintertemperatur von $Al_2O_3$-Submikrometerpulvern wird insbesondere durch Dotierungen von mehr als 1 %, die beim Sintern flüssige Phasen bilden, auf 1200 °C und weniger reduziert, jedoch bleiben die Festigkeiten auf dem für traditionellen Sinterkorund üblichen Niveau von ca. 400 MPa (L.A. Xue u.a., J. Am. Ceram. Soc., (1991),S.2011-2013), und die dabei häufig entstehenden Korngrenzenphasen bringen ungünstige Hochtemperatureigenschaften mit sich.

Obwohl der Zusammenhang von Defektstruktur und Festigkeit spröder Festkörper seit langem gut bekannt ist, beschränken sich die meisten Untersuchungen auf die rein qualitative Feststellung relevanter Defektypen; schon eine Charakterisierung nur relativer Defektgrößenverteilungen (H.E. Exner u.a., Mater. Sci. Eng. 16(1974), S. 231-238) ist selten. Zur Technologie-Abhängigkeit der eigentlich wichtigen absoluten Fehlerhäufigkeit pro Volumen bzw. analysierte Fläche, wie beispielhaft für Glas untersucht (J.R. Matthews u.a., J. Am. Ceram. Soc. 59(1976), S.304-308), ist für $Al_2O_3$-Keramik nichts bekannt. Überhaupt keine Erfahrungen gibt es zur Auswirkung solcher Defekte auf die Vickers-Härte.

Hinsichtlich der Bewertung der Härte von so hergestellten $Al_2O_3$-Keramiken ist zunächst der Einfluß der angewandten Prüfbelastung des Indenters zu berücksichtigen. Die Vickers-Härte von $Al_2O_3$ ist für ein- wie polykristallines Material in komplexer Weise von der Last abhängig (A. Krell, Kristall und Technik (1980)12,1467-74). Typisch ist folgendes Werkstoffverhalten:

- relativ geringer Lasteinfluß bei größerer Prüflast $\geq$ 50 N,
- sinkende Belastung erzeugt zunächst steigende Härtewerte,
- bei weiter reduzierter Prüflast < 1 bis 5 N kann die Härte erneut sinken.

Für die Härtemessung nach Knoop gilt Ähnliches, nur daß zwischen den zahlenmäßigen Ergebnissen nach Knoop und nach Vickers bestimmte systematische Abweichungen bestehen.

Nach dem im folgenden beschriebenen Stand der Technik für die mittels bekannter Verfahren hergestellten $Al_2O_3$-Sinterprodukte aus Korundpulver gilt die seit langem gesicherte Erkenntnis, daß unabhängig von der jeweiligen Prüflast eine Korngrößenreduzierung im Bereich von 2 - 0,2 $\mu$m keine Härtesteigerung über die bekannten Obergrenzen von HV10 $\approx$ 2000 (Kleinlasthärte) bzw. HV0,2 $\approx$ 2500 (Mikrohärte) hinaus ermöglichen kann (S.D.Skrovanek u.a., J.Am.Ceram.Soc. (1979)3/4, 215-216). Aus den dargelegten Gründen ist dabei ein Vergleich von Härteangaben der Literatur nur dann aussagekräftig, wenn die Lastabhängigkeit der Härte untersucht und die Prüfbedingungen dazu angegeben sind. Dies gilt insbesondere deshalb, weil der Einfluß der Prüflast selbst innerhalb einer Werkstoffgruppe, wie z.B. Sinterkorund, in Abhängigkeit von Dotierungen, Restporosität und vor allem vom Oberflächenzustand extrem unterschiedlich ist. Infolge der vielfältigen und nur selten spezifizierten Angaben im Stand der Technik schwanken die Angaben zum Korngrößeneinfluß auf die Mikrohärte und Kleinlast- oder Makrohärte in weiten Grenzen:

Mikrohärte HV (im Bereich HV0,1-HV0,5, d.h. im Bereich des möglichen Maximums der Härte-Last-Kurve)

- für Einkristalle: 2300 - 2700
  (A.G.Evans u.a.,J.Am.Ceram.Soc. (1976)7/8,371-372)
- für Sinterkorund mit D $\approx$ 2$\mu$m
  bei relativer Dichte $\rho \geq$ 99 %: 2000 - 2600
  (S.D.Skrovanek u.a.,J.Am.Ceram.Soc. (1979)3/4,215-216)

Vickers-Kleinlast- und Makrohärte (Prüflast $\geq$ 10 N)

- für Einkristalle: 1400 - 1700
  (A.Krell, Kristall und Technik (1980)12,1467-1476)
- für Sinterkorund mit D $\approx$ 2 $\mu$m
  bei relativer Dichte $\rho \geq$ 99 %: 1650 - 1850
  (A.Krell, Kristall und Technik (1980)12,1467-1476)
- für Sinterkorundprodukte mit D $\approx$ 0,45 $\mu$m, pulvertechnologisch hergestellt,
  bei relativer Dichte $\rho$ = 98 -98,5 %: 1900 - 2000
  (G.Riedel u.a.,Silicates Industriels (1989)1/2,29-35).

Spezielle Untersuchungen von Skrovanek u.a. zur Korngrößenabhängigkeit der Härte in nahezu vollständig dichten heißgepreßten Sinterkorunden mit abgestuft-abnehmender Korngröße zeigten einen Härteanstieg proportional $D^{-1/2}$ nur bis in den Bereich von 3 - 4 $\mu$m. Nach allgemeiner Auffassung ist dies durch die zunehmende Behinderung der Versetzungsbewegung in den kleineren Körnern bedingt. Weitere Korngrößenreduzierungen bis 1,7 $\mu$m erbrachten jedoch keinen weiteren Anstieg der Härte; die Härte blieb in diesem Bereich kleiner Korngrößen konstant. Diesem Befund wurde bisher nie widersprochen, weil nach allgemeiner Auffassung eine mit verringerter Korngröße weiter zunehmende Behinderung der Versetzungsbewegung und steigende Härte dann nicht mehr zu erwarten ist, wenn die Korngrößen in den Größenbereich der Versetzungen kommen. Da typische, mittels Transmissionselektronenmikroskopie in Korund beobachtete Größen von Versetzungen (Versetzungsloops) im Mikrometerbereich liegen, galt die Verringerung des Korngrößeneffektes der Härte bei Korngrößen von weniger als 3 - 4 $\mu$m als so selbstverständlich, daß diese Frage nicht tiefer untersucht wurde.

Der Nachteil der nach dem Stand der Technik bekannten $Al_2O_3$-Sinterprodukte hergestellt aus Korundpulver besteht darin, daß keine hochgradig defektarmen $Al_2O_3$-Sinterprodukte mit sehr hoher Härte bzw. hoher Härte und Festigkeit bekannt und herstellbar sind. Ohne daß bisher schlüssige Lösungen der Probleme bekannt geworden wären, werden aktuelle Entwicklungen international auf zwei für erfolgversprechend gehaltene Richtungen konzentriert (T.J. Carbone, S. 107 in: L.D. Hart (Hrsg.), "Alumina Chemicals Science and Technology Handbook", The Am. Ceram. Soc., Westerville, Ohio, 1990):

(1) Verbesserung der Sol/Gel-Technologien mit Keimzusätzen (z.B. G.L. Messing und M. Kumagai in Bull. Am. Ceram. Soc. 73(1994)88-91),

(2) die Entwicklung und Verwendung von als ideal empfundenen Pulvern extrem einheitlicher Korngröße ("monosized", "uniform-sized") wie von K. Yamada in "Alumina Chemicals Science and Technology Handbook" beschrieben (Hrsg. L.D. Hart, The Am. Ceram. Soc., Westerville, Ohio, 1990, Seite 564).

EP-A-0 519 159 offenbart polykristalline, gesinterte Schleifkörner auf Basis von $\alpha$-$Al_2O_3$. Die relative Härte dieser Schleifkörner wird lediglich als Mikrohärte bei einer Prüflast von 500g (5 N) gemessen. Angaben über Defekthäufigkeiten werden nicht gemacht.

EP-A-0 408 771 offenbart Schleifmaterialien auf Korundbasis, die lediglich Dichten deutlich unterhalb 98,5% der theoretischen Dichte erreichen. Es werden auch hier lediglich Mikrohärtemessungen durchgeführt.

Der Erfindung liegt die Aufgabe zugrunde, $Al_2O_3$-Sintermaterialien mit verbesserten mechanischen Eigenschaften, insbesondere mit hoher Härte und/oder Festigkeit zu schaffen.

Die Aufgabe wird durch die in den unabhängigen Ansprüchen angegebene Erfindung gelöst. Vorteilhafte Weiterbildungen finden sich in den abhängigen Ansprüchen.

Die erfindungsgemäßen $Al_2O_3$-Sintermaterialien mit einem $Al_2O_3$-Gehalt von 95 bis 100 Vol-% und einer Vickers-Kleinlast-Härte $\geq$ 2000 bei einer Prüflast von 10 N bis 100 N (HV1 bis HV10), weisen eine relative Sinterdichte von $\rho \geq$ 98,5 %, Gefüge mit mittleren Korngrößen von $\leq$ 1,5 $\mu$m und Inhomogenitäten einer Häufigkeit von < 50 x $10^{+9}$ $m^{-2}$ auf.

Die Inhomogenitäten gehören einer oder mehrerer der folgenden Kategorien an:

a) Risse und/oder poröse Gebiete entlang der Grenzen von Pulveraggregaten/Pulveragglomeraten,

b) nestartige Gefügebereiche aufgelockerter, mit Poren durchsetzter Gefügestruktur,

c) Poren mit einem die doppelte Gefügekorngröße übersteigenden Durchmesser.

Es können ggf. Inhomogenitäten der folgenden Kategorie zusätzlich berücksichtigt werden:

d) Körner > 10$\mu$m und/oder mehr als 10 $\mu$m große Agglomerate einzelner Körner, deren Durchmesser die fünffache mittlere Korngröße übersteigt.

Zusätzlich können ggf. bei den Inhomogenitäten der Kategorie c) alle Poren mit einem Durchmesser $\geq$ 0,3 $\mu$m berücksichtigt werden.

Vorteilhafterweise ist die Häufigkeit der Inhomogenitäten $\leq$ 20 x $10^{+9}$ $m^{-2}$. Bei Sinterkorund des Standes der Technik beträgt dagegen die Häufigkeit der in Anspruch 1 definierten Inhomogenitäten mehr als 50 x $10^{+9}$ $m^{-2}$.

Vorteilhafterweise können mit dem erfindungsgemäßen Verfahren sogar Sintergefüge mit mittleren Korngrößen unterhalb von 0,8 $\mu$m erhalten werden.

Vorteilhafterweise weist das Sintermaterial ein Gefüge mit einer überwiegend regellosen Orientierung der Kristallite auf.

Bei einer weiteren Ausführungsform der Erfindung wird Wert gelegt auf eine Erzielung sowohl einer verhältnismäßig hohen Härte als auch auf eine hohe Biegebruchfestigkeit. Das zusätzliche Erfordernis einer hohen Biegebruchfestigkeit bei dieser Ausführungsform erfordert ein Sintermaterial, das als qualifizierendes Merkmal auch eine bestimmte Größenverteilung der Defekte bzw. Inhomogenitäten aufweist. Dies wiederum erfordert die Charakterisierung des erfindungsgemäßen Sintermaterials anhand einer dimensionslosen Defektdichte, definiert als Summe der Defektgrößen-Quadrate pro analysierte Fläche, wie sie ursprünglich zur Bewertung von Mikrorißdichten eingeführt worden ist (A. Krell u.a., J. Mater. Sci. (1987)9, 3304-08). Dabei wird als Defektgröße die maximale in einer beliebigen Richtung erkennbare Ausdehnung des Defektes in der analysierten Ebene verwendet. Bei keramischen Materialien des Standes der Technik beträgt die Häufigkeit solcher Inhomogenitäten mehr als 30 x $10^{-3}$. Die weitere erfindungsgemäße Ausführungsform ist gekennzeichnet durch einen Gehalt von 95 bis 100 Vol-% $Al_2O_3$ und eine Vickers-Kleinlast-Härte $\geq$ 1750 bei einer Prüflast von 10 N bis 100 N (HV1 bis HV10) und einer Biegebruchfestigkeit $\geq$ 800 MPa, wobei das Sintermaterial eine relative Sinterdichte von $\rho \geq$ 98,5 %, ein Gefüge mit einer mittleren Korngröße von $\leq$ 2 $\mu$m und eine dimensionslose Defektdichte von < 30 x $10^{-3}$ aufweist, wobei die Defekte einer oder mehrerer der folgenden Kategorien angehören:

a) Risse und/oder poröse Gebiete entlang der Grenzen von Pulveraggregaten/Pulveragglomeraten,

b) nestartige Gefügebereiche aufgelockerter mit Poren durchsetzter Gefügestruktur,

c) Poren mit einem die doppelte mittlere Gefügekorngröße übersteigenden Durchmesser,

d) Körner > 10 $\mu$m und/oder mehr als 10 $\mu$m große Agglomerate einzelner Körner mit einem die fünffache mittlere Korngröße übersteigendem Durchmesser.

Auch das Gefüge desjenigen Sintermaterials, das hohe Härte mit hoher Biegebruchfestigkeit kombiniert, weist vorteilhafterweise eine überwiegend regellose Orientierung der Kristallite auf.

Die erfindungsgemäßen Sinterprodukte können außer Korund ($\alpha$-$Al_2O_3$) noch bis zu 5 Vol.-% anderer Substanzen enthalten, solange die oben genannten zulässigen Grenzen der relativen Dichte, der Häufigkeit der genannten Inhomogenitäten bzw. der dimensionslosen Defektdichte dadurch nicht verletzt werden. Die Bruchzähigkeiten ($K_{Ic}$) der erfindungsgemäßen Produkte können das für Sinterkorund typische, je nach Meßverfahren zwischen ca. 3 und 4,5 MPa$\sqrt{m}$ liegende Niveau erheblich übertreffen. Für die Realisierung der Erfindung ist dies jedoch keine Bedingung. Grundsätzlich ist bei der Ermittlung von Bruchzähigkeiten zu beachten, daß insbesondere das verwendete Meßverfahren einen starken und quantitativ kaum beschreibbaren Einfluß auf den ermittelten Meßwert hat, so daß Meßwerte häufig nicht miteinander vergleichbar sind.

Ein Verfahren zur Herstellung eines erfindungsgemäßen $Al_2O_3$-Sintermaterials umfaßt die folgenden Schritte:

a) Überführen eines $\alpha$-$Al_2O_3$-Pulvers mit einer mittleren Korngröße $d_{50} \leq 0,30$ $\mu$m und einer chemischen Reinheit von $\geq$ 99,9% $Al_2O_3$ in einer Flüssigkeit in eine stabile Suspension dispergierter Partikel mittels gleichzeitiger und/oder sequentieller Anwendung von mindestens zwei unterschiedlichen Dispergierverfahren,

b) Herstellung eines ungesinterten Vorprodukts mit einer relativen Dichte von $\rho \geq 55$ % mittels Formgebung,

c) Wärmebehandlung und Sinterung des Vorprodukts.

Das erfindungsgemäße Verfahren erfordert nicht die Verwendung sehr teurer Pulver einheitlicher ("monosized", "uniform-sized") Korngröße, es kann vielmehr vorteilhaft mit einem sehr viel kostengünstigeren $\alpha$-$Al_2O_3$-Pulver der folgenden Korngrößenverteilung ausgeführt werden: $d_{16} > 0,065$ $\mu$m, $d_{50} \leq 0,30$ $\mu$m, $d_{84} \leq 0,45$ $\mu$m. Dies ist ein besonderer Vorteil der Erfindung, da eine kostengünstigere Herstellung entsprechend hochwertiger Sintermaterialien als im Stand der Technik möglich ist. Wesentlich für den Erfolg des erfindungsgemäßen Herstellungsverfahrens ist die Herstellung einer stabilen (und besonders homogenen) Suspension der dispergierten Partikel durch gleichzeitige und/oder sequentielle Anwendung von mindestens zwei unterschiedlichen Dispergierverfahren.

Vorteilhafterweise weist das in Schritt a) eingesetzte $\alpha$-$Al_2O_3$-Pulver einen nach der BET-Methode bestimmte spezifische Oberfläche von 10 bis 17 $m^2$/g auf.

Die oben angegebene Korngrößenverteilung des eingesetzten $\alpha$-$Al_2O_3$-Ausgangspulvers ist für den Erfolg der Erfindung von besonderer Bedeutung. Größere mittlere Korngrößen als zulässig, höhere Grobkornanteile ($d_{84}$ zu höheren Werten verschoben) und entsprechend niedrigere spezifische Oberflächen verringern die Sinteraktivität, erhöhen die erforderliche Sintertemperatur und führen so zu unzulässig groben Gefügen. Kleinere mittlere Korngrößen bzw. höhere Feinstkornanteile ($d_{16}$ zu niedrigeren Werten verschoben) verursachen Probleme bei der anschließenden Formgebung und Sinterung: die dadurch verringerte Gründichte (d.h. die Dichte der ungesinterten Produkte) wie auch eine erhöhte Defekthäufigkeit erfordern ebenfalls erhöhte Sintertemperaturen, so daß gröbere und mit mehr Inhomogenitäten und Defekten behaftete Sinterprodukte entstehen. Eine verringerte Reinheit des Pulverrohstoffes unterhalb des erfindungsgemäß angegebenen Werts führt zumindest lokal zur Bildung flüssiger Phasen beim Sintern, so daß die hergestellten Gefüge amorphe Korngrenzenphasen, Ausscheidungen, Mikroporen und andere unerwünschte Mikrodefekte enthalten. Selbst wenn amorphe Phasen nicht nachweisbar sind, führt eine Verringerung der erfindungsgemäß einzuhaltenden Pulverreinheit zu unkontrollierbaren Kornwachstumsprozessen beim Sintern.

Zur Herstellung eines $\alpha$-$Al_2O_3$-Sintermaterials, bei dem nicht ausschließlich Wert auf eine besonders hohe Härte gelegt wird, sondern bei dem man eine hohe Biegebruchfestigkeit unter Inkaufnahme einer etwas verringerten Härte anstrebt, können in einer Abwandlung des erfindungsgemäßen Herstellungsverfahrens folgende Schritte zur Anwendung kommen:

a) Überführen eines $\alpha$-$Al_2O_3$-Pulvers mit einer durch die Parameter $d_{16} > 0,065$ $\mu$m, $0,2$ $\mu$m $\leq d_{50} \leq 0,4$ $\mu$m, $0,45$ $\mu$m $\leq d_{84} \leq 0,8$ $\mu$m bestimmten Korngrößenverteilung und einer chemischen Reinheit von $\geq$ 99,9% $Al_2O_3$ in einer Flüssigkeit in eine stabile Suspension dispergierter Partikel mittels gleichzeitiger und/oder sequentieller Anwendung von mindestens zwei unterschiedlichen Dispergierverfahren,

b) Herstellung eines ungesinterten Vorprodukts mit einer relativen Dichte von $\rho \geq 55$ % mittels Formgebung,

c) Wärmebehandlung und Sinterung des Vorprodukts.

Im Unterschied zum erstgenannten Verfahren ist hier ggf. eine geringfügig höhere mittlere Korngröße und ein etwas höherer Grobkornanteil zulässig. Es kann also ein noch preiswerteres Ausgangsmaterial verwendet werden. Größere mittlere Korngrößen als zulässig, höhere Grobkornanteile ($d_{84}$ zu höheren Werten verschoben) und entsprechend niedrigere spezifische Oberflächen verringern auch hier die Sinteraktivität, erhöhen die Sintertemperatur in unzulässiger Weise und führen so zu Gefügen, die für die Realisierung der angeführten mechanischen Parameter zu grobkörnig sind. Höhere Feinstkornanteile ($d_{16}$ zu niedrigeren Werten verschoben) und eine verringerte Reinheit des Pulverrohstoffes verursachen auch hier dieselben Probleme wie oben diskutiert.

Das bei dieser Abwandlung des erfindungsgemäßen Herstellverfahrens als Ausgangsmaterial eingesetzte $\alpha$-$Al_2O_3$-Pulver weist vorteilhafterweise eine nach der BET-Methode bestimmte spezifische Oberfläche von 8 - 17 $m^2$/g auf.

Die im Rahmen der Erfindung eingesetzten Sinterverfahren können drucklos sein oder mit Druck arbeiten (z. B.

Heißpressen oder heißisostatisches Pressen). Ein besonderer Vorteil der Erfindung liegt aber darin, daß man $\alpha$-$Al_2O_3$-Materialien hoher Härte und/oder Festigkeit auch bei der Anwendung druckloser Sinterverfahren erhält.

In diesem Zusammenhang sei darauf hingewiesen, daß druckloses Sintern an Luft im Gegensatz zu Heißpressen oder heißisostatischem Pressen (HIP) aufgrund der Unterschiede der Umgebungsatmosphäre (insbesondere des Sauerstoffpartialdrucks) zu erheblichen Strukturunterschieden führt, die ihrerseits Diffusionskoeffizienten und davon abhängige mechanische, optische und elektrische Eigenschaften verändern (S.K. Mohapatra u.a., J. Am. Ceram. Soc. (1978), Heft 3/4, 106-109; S.K. Mohapatra u.a., J. Am. Ceram. Soc. (1979), Heft 1/2, 50-57).

Beim Heißpressen oder heißisostatischem Pressen wird üblicherweise in einer Schutzgasatmosphäre (Argon) gearbeitet, es liegt also ein verminderter Sauerstoffpartialdruck vor, andererseits entsteht aufgrund der im Rahmen der Preßverfahren verwendeten kohlenstoffhaltigen Materialien ein CO-Partialdruck, der reduzierend wirkt, so daß Sauerstoffleerstellen als Punktdefekte über das gesamte Korundgitter verteilt entstehen, die zu den obengenannten geänderten Eigenschaften führen. U.a. kommt es zu einer grau-dunklen Färbung des Korunds im Gegensatz zu dem typischen hellen Erscheinungsbild von durch druckloses Sintern an Luft hergestellten $Al_2O_3$-Sintermaterialien (T. Nagatome u.a., J. Ceram. Soc. Japan, Int. Ed. (1994), Heft 1, 147-151).

Für beide Varianten des Herstellverfahrens gilt, daß vorteilhafterweise die in Schritt a) angewendeten Dispergierverfahren aus der folgenden Gruppe ausgewählt werden:

- mechanische Rühreinrichtung,
- Beaufschlagung mit Ultraschall,
- Anwendung einer Kugelmühle.

Bei der Anwendung einer Kugelmühle kommt es weniger auf deren Mahlwirkung als vielmehr auf deren das Mahlgut mischende und damit dispergierende Wirkung an, auch werden ggf. entstandene Pulveragglomerate zerstört. Es sei angemerkt, daß diese Aufzählung der Dispergierverfahren nicht abschließend ist und andere geeignete Dispergierverfahren ebenfalls verwendet werden können. Die Erfindung hat allerdings erkannt, daß die Anwendung eines einzigen Dispergierverfahrens in keinem Fall zur Durchführung der erfindungsgemäßen Verfahren und zur Herstellung erfindungsgemäßen $\alpha$-$Al_2O_3$-Materialien ausreichend ist.

Das erfindungsgemäß zu verwendende Ausgangspulver definierter Beschaffenheit wird in einer Flüssigkeit, vorzugsweise in destilliertem oder deionisiertem Wasser mit pH-Werten im Bereich von 3 - 7, vorzugsweise 4 - 6, weiter vorzugsweise um 5, dem für die Dispergierung von $\alpha$-$Al_2O_3$-Pulver übliche Dispergierhilfsmittel in Form von Mineralsäuren, Carbonsäuren, Polycarbonsäuren oder anderen Polyelektrolyten zugesetzt werden können, in eine stabile hochdisperse Suspension überführt. Dies erfolgt durch eine synergetisch wirkende Kombination unterschiedlicher Dispergierverfahren, z.B. durch die bereits genannte kombinierte Anwendung von hochtourigem Rührwerk, Ultraschall oder von Kugelmühlen. Durch diese Intensiv-Dispergierung wird eine hohe Stabilität der im wesentlichen agglomeratfreien Suspension bereits im pH-Bereich von 3 - 7 erreicht, während nach verbreiteter Auffassung für die hier ausschließlich in Frage kommenden feindispersen Pulver ein ausreichender Dispersionsgrad erst bei pH < 3 erreicht wird (T. Kimura u.a., Sci. Sintering (1990) 2, 59-64). Solch niedrige pH-Werte bringen aber neben der korrosiven Belastung der Aggregate und Behälter auch zusätzliche Nachteile für die Schlickerqualität mit sich (z.B. kann, bedingt u.a. durch Lösungsprozesse, die Viskosität steigen und eine Beschränkung auf niedrige Feststoffgehalte erzwingen).

Zur Intensivierung des Verdichtungsprozesses und zur Steuerung der Gefügeentwicklung beim Sintern können der Dispersion im Rahmen des erfindungsgemäßen Verfahrens chemische Substanzen zugesetzt werden, welche üblicherweise Elemente wie z.B. Mg oder auch Li, Ba, Sr, Zn, Fe, Co, Ni, Cr, Zr, Ti, Si, Y, Ce, La oder Y bis zu einer Gesamtkonzentration von 0,5 Ma.-% (bezogen auf das $Al_2O_3$) enthalten. Das erfindungsgemäß zu verwendende hochdisperse Ausgangspulver definierter Beschaffenheit führt allerdings zu einer derartigen Struktur der grünen Zwischenprodukte, daß beim Sintern die kornwachs- tumshemmende Wirkung von Substanzen wie Mg gering (und weitgehend unnötig) ist; sie ist aber in vielen Fällen noch nachweisbar.

Der so hergestellte Schlicker wird entweder unter Anwendung von Druck oder auch durch drucklose Gießverfahren zu einem zunächst ungesinterten Vorprodukt verarbeitet, das eine relative Dichte > 55 % aufweist.

Die anwendbaren Verfahren unter Verwendung von Druck gliedern sich in 2 Gruppen: bindemittelfreie Verfahren wie Druckfiltration und solche, bei denen dem Schlicker während der Intensiv-Dispergierung noch Binde- bzw. Preßhilfsmittel zugegeben werden (kaltisostatisches Pressen, Strangpressen, Spritzgießen).

Besonders hohe Härten werden erreicht, wenn die Korngröße der erfindungsgemäßen $Al_2O_3$-Sinterprodukte mit einer relativen Dichte von $\geq$ 98,5% vorteilhafterweise auf weniger als 0,8 $\mu$m reduziert und gleichzeitig ein besonders homogener, Inhomogenitäten in Form von Porenstrukturen jeglicher Art vermeidender Gefügeaufbau erzielt wird. Ein besonders vorteilhaftes Verfahren zur Herstellung solcher Sinterprodukte besteht in der Druckfiltration von erfindungsgemäß hergestellten Schlickern wie oben beschrieben, wobei die Schlicker einen Feststoffgehalt von mindestens 60 Ma.-% aufweisen, gefolgt von kaltisostatischem Pressen des zuvor auf eine Restfeuchte von günstigerweise 0,5 - 3 % getrockneten Körpers. Der Druck bei der Druckfiltration liegt zwischen 0,3 und 20 MPa, vorteilhafterweise im Bereich um 3,5 ($\pm$ ca. 2) MPa, für das Nachverdichten mittels kaltisostatischen Pressens ist ein Druckbereich $\geq$ 200 MPa aus-

reichend. So hergestellte Formkörper erreichen im Sinterprozeß schon bei Temperaturen ab 1200 - 1250°C relative Dichten von 99 %, die mittleren Gefügekorngrößen können in den Bereich von ca. 0,4 $\mu$m reduziert werden. Geschliffene Produkte dieser Art weisen z.B. Härten von HV1 > 2300 auf, die erreichten Festigkeiten liegen im Bereich von ca. 650 - 750 MPa.

Ein besonders wirtschaftliches Verfahren ist dadurch gegeben, daß erfindungsgemäße defektarme $\alpha$-Al$_2$O$_3$-Sinterkörper mit Vickers-Kleinlast-Härten > 2000 bei einer relativen Sinterdichte von $\geq$ 98,5 % und mittleren Korngrößen $\leq$ 1,5 $\mu$m, insbesondere auch mit mittleren Korngrößen im Bereich von 0,6 - 0,9 $\mu$m, bei erfindungsgemäßer, oben beschriebener Herstellung eines Schlickers aus dispergiertem Korundpulver definierter Beschaffenheit nach Zusatz eines Preßhilfsmittels und Überführung des Schlickers in ein Granulat durch eine Formgebung von Grünkörpern mittels Anwendung des in der keramischen Industrie verbreiteten kaltisostatischen Pressens bei relativ niedrigem Druck von $\geq$ 200 MPa hergestellt werden; vorteilhaft ist ein (z.B. uniaxiales) Vorpressen bei niedrigem Druck von 20 - 200 MPa, besonders vorzugsweise im Bereich um 40 ($\pm$ ca. 10) MPa. Voraussetzung ist die Überführung des Schlickers in ein Granulat, welches sich beim Pressen so verhält, daß im grünen Formkörper nahezu keine Agglomerate von Pulverteilchen auftreten. Die Optimierung des Granulats erfolgt über eine gegenseitige Anpassung der zugesetzten, allgemein bekannten Bindemittel und des Trocknungs- und Granulierregimes.

Ein besonders vorteilhaftes und formuniverselles Verfahren zur Herstellung erfindungsgemäßer, extrem defektarmer Al$_2$O$_3$-Sinterprodukte mit hohen Festigkeiten von mehr als 800 MPa und mit hohen Vickers-Kleinlast-Härten von mehr als 1750 bei einer relativen Dichte von $\geq$ 98,5 % und mittleren Gefüge-Korngrößen von $\leq$ 2 $\mu$m besteht im drucklosen Gießen der Schlicker und anschließender Konsolidierung durch Koagulationsprozesse, durch in-situ Polymerisation eines zugesetzten Monomers oder Ähnliches. Im Falle der Konsolidierung durch Polymerisation werden der auf einen Feststoffgehalt von > 60 Ma.-%, typischerweise von 70 - 85 Ma.-%, eingestellten wäßrigen Al$_2$O$_3$-Dispersion ein polymerisierbares Monomer, z.B. Acrylamid oder Hydroxyethylacrylat, und ein die Polymerisation auslösender Initiator, z.B. Ammonium-Persulfat zugesetzt. Im Rahmen der erfindungsgemäßen Verfahrensweise zur Herstellung erfindungsgemäßer, außerordentlich defektarmer Sinterprodukte aus einem hochdispersen Korundpulvers der beschriebenen definierten granulometrischen Beschaffenheit entscheidend ist einerseits die besonders sorgfältige Intensiv-Dispergierung des Korundpulvers mit dem Monomer wie auch die anschließende, noch vor Beginn der Polymerisation vorzunehmende (vorteilhafterweise durch Ultraschall unterstützte) Entgasung des Schlickers bei einem reduzierten Druck von 100 - 300 mbar.

Nach dem Entgasen wird der Schlicker in Formen gegossen und in Stickstoff-Atmosphäre entweder durch Zusatz eines Katalysators oder durch Temperaturerhöhung auf 55 - 80 °C polymerisiert.

Die Trocknung der Formkörper kann für alle Formgebungsvarianten über 2 - 3 Tage an Luft und anschließend im Trockenschrank bei 70 - 110 °C erfolgen; die erforderliche Dauer des Trocknungsprozesses verkürzt sich mit sinkendem Feuchtegehalt der Produkte. Besonders vorteilhaft ist die Trocknung in einem Trockenschrank mit Temperatur- und Feuchte-Regelung, wobei die Aufheizrate bis 90 °C 1-2 K/h beträgt und gleichzeitig eine Absenkung der Raumfeuchte von 60 - 80 % auf 15 - 20 % erfolgt. Nach einer Haltezeit von 4 - 6 h bei 90 °C werden die Produkte unter Beibehaltung der niedrigen Raumfeuchte auf Raumtemperatur abgekühlt.

Gegebenenfalls kann eine kaltisostatische Nachverdichtung vorgesehen sein. Der Grünkörper sollte dann vor dieser Nachverdichtung auf eine Restfeuchte von 0,5 - 3 % eingestellt werden.

Die relative Dichte der getrockneten Proben liegt bei $\geq$ 55 % der theoretischen Dichte, vorteilhafterweise bei $\geq$ 60 %.

Die getrockneten Proben werden einer thermischen Behandlung bei 700 - 900 °C in Luft unterzogen, wobei die Aufheizrate bei 0,1 bis 0,3 K/min liegt und die Haltezeit der thermischen Behandlung 1 - 4 h beträgt.

Die Sinterung erfolgt bei einer Temperatur zwischen 1200 und 1500 °C in Luft, wobei die Aufheizrate 1 - 3 K/min und die Haltezeit bei Sintertemperatur 0,5 - 8 h beträgt.

So hergestellte Produkte können hohe Festigkeiten auch noch bei einem relativ groben Gefüge mit Korngrößen zwischen 1 und 2 $\mu$m erreichen. Zum Beispiel wurde eine mittlere Biegebruchfestigkeit von 862 MPa bei einer mittleren Korngröße von 1,31 $\mu$m gemessen.

Der auf dem Gebiet dieser Gießtechnik durch die erfindungsgemäßen Verfahrensschritte erreichte sehr große Festigkeitsgewinn gegenüber dem bisher 400 MPa nicht übersteigenden Stand der Technik (A.C. Young, u.a., J. Am. Ceram. Soc. (1991)3, 612-618; R. Wäsche u.a., cfi/Ber.DKG (1995) 1/2, 24-27) ist ein besonders überraschendes Ergebnis der Erfindung.

Angesichts des umfangreichen bekannten Standes der Technik und der bisherigen Selbstverständlichkeit, mit der Aussagen wie die von Skrovanek u.a. zu einer unterhalb von 3 - 4 $\mu$m Korngröße nicht weiter steigenden Härte von Sinterkorund akzeptiert wurden, war es außerordentlich überraschend, daß Sinterprodukte mit einer Vickers-Kleinlast-Härte $\geq$ 2000 bei einer Prüflast von 10 N bis 100 N (HV1 bis HV10) bzw. mit einer Vickers-Kleinlast-Härte $\geq$ 1750 und einer Biegebruchfestigkeit $\geq$ 800 MPa angegeben und erzeugt werden konnten durch das Herstellen von Gefügen einer mittleren Korngröße von $\leq$ 1,5 bzw. $\leq$ 2 $\mu$m und einer relativen Sinterdichte von $\rho \geq$ 98,5 %.

Die Untersuchungen, wie auch der bekannte Stand der Technik haben ergeben, daß allein eine wie erfindungsgemäß genannte relative Sinterdichte oder kleine Korngröße in einem Sinterprodukt nicht ausreichend für eine höhere Härte sind. Erst die Kombination einer Gefügekorngröße ≤ 1,5 µm mit einer relativen Sinterdichte ρ ≥ 98,5 % und mit der Vermeidung von Inhomogenitäten und Defekten in einer festgelegte Obergrenzen übersteigenden Häufigkeit und Dichte im Sinterprodukt führen zu dieser hohen Vickers-Kleinlast-Härte und Festigkeit.

Ein besonderer Vorteil des erfindungsgemäßen Verfahrens besteht darin, daß es für in der keramischen Industrie verbreitete Technologien nutzbar ist, welche auf diese Weise die Herstellung auch größerer hochgradig defektarmer Formkörper definierter geometrischer Abmessungen mit hoher Härte ermöglichen.

Die genannten Eigenschaften der neuartigen $Al_2O_3$-Sinterprodukte aus Korundpulver ermöglichen einen vorteilhaften Einsatz unter sehr unterschiedlichen technischen Beanspruchungen. Verschiedene Beispiele sind in den Ansprüchen 29 bis 34 angegeben. So ermöglicht ihre sehr hohe Härte bei gleichzeitig hoher Festigkeit eine erhöhte Zuverlässigkeit beim Einsatz von Humanimplantaten, verbessert die Verschleißresistenz von Verschleißprodukten (wie z.B. von Schneid- oder anderen Werkzeugen bzw. Teilen davon, von verschleißresistenten Bauteilen in Maschinen, Armaturen, Pumpen, Lagern, von Fadenführern, Leit- und Umlenkelementen, von Substraten für mikroelektronische Anwendungen, von Disketten für Datenspeicher (computer disks) und erhöht die mit gesinterten Korundschleifmitteln dieser Art erreichte Abtragleistung. Die erfindungsgemäßen Materialien können vorteilhaft in Form von gesinterten Körnungen (bspw. gebunden auf Unterlagen oder in Schleifkörpern) als Schleif- oder Poliermittel verwendet werden.

Im weiteren wird die Erfindung an mehreren Ausführungsbeispielen erläutert. Im Beispiel 1 ist die beste Ausführungsform für ein Produkt sehr hoher Härte gemäß den Ansprüchen 1 bis 5 angegeben, Beispiel 4 zeigt ein Produkt besonders günstiger Ausführung zur Verbindung hoher Härte und Festigkeit gemäß den Ansprüchen 6 und 7.

Beispiel 1

150 g einer feindispersen α-Tonerde mit engem Korngrößenspektrum Taimicron TM-DAR ($d_{16}$= 0,13 µm, $d_{50}$= 0,19 µm, $d_{84}$= 0,25 µm, BET= 14 $m^2$/g) wurden mittels eines hochtourigen Rührwerkes (5000 U/min) in einer wäßrigen Lösung aus 120 ml destilliertem Wasser, 9 ml 10%-ige Polyvinylalkohol-Lösung und 3 ml Glyzerin 30 min dispergiert.

Die Dispersion wurde anschließend in einer Labor-Rührwerkskugelmühle mit YTZP-Mahlgarnitur 1 h bei einer Rührerdrehzahl von 1000 U/min gemahlen und dadurch homogenisiert und weiter dispergiert und danach einer Gefriertrocknung unterzogen.

Der auf eine Restfeuchte von < 2 % gefriergetrocknete Versatz wurde durch ein 300 µm-Sieb gestrichen und bei 30 MPa uniaxial zu einem Formkörper vorverdichtet. Der Formkörper wurde anschließend in einer dünnen elastischen Hülle kaltisostatisch bei 700 MPa gepreßt. Nach Trocknung bei 80 °C betrug die erreichte relative Dichte 61,0 % der theoretischen Dichte.

Der Formkörper wurde bei 800 °C in Luft vorgebrannt (Aufheizrate 0,3 K/min, 1 h Haltezeit bei 800 °C) und anschließend in Luft bei 1400 °C drucklos gesintert (Aufheizrate 2 K/min, Haltezeit 2 h bei Sintertemperatur, Ofenabkühlung).

Die Defektanalyse erfolgte mittels Rasterelektronenmikroskop an polierten und thermisch bei 1300 °C geätzten Oberflächen. Defekte im Sinterprodukt traten typischerweise in Form einzelner Poren der Größe 0,3 - 30 µm mit einer geringen Häufigkeit von 8 x $10^{+9}$ $m^{-2}$ auf; die die Defektgrößenverteilung berücksichtigende dimensionslose Defektdichte betrug 10 x $10^{-3}$. Nach dem Stand der Technik durch Trockenpressen und Sintern aus demselben Rohstoff hergestellte Sinterkörper zeigten dagegen eine Häufigkeit solcher Gefügeinhomogenitäten von 40 x $10^{+9}$ $m^{-2}$ bzw. eine dimensionslose Defektdichte von 96 x $10^{-3}$. Bei Begrenzung der Auswertung auf Poren ≥ 1,3 µm (= doppelte mittlere Korngröße) wurde im erfindungsgemäßen Produkt eine wesentlich geringere Häufigkeit von 0,85 x $10^{+9}$ $m^{-2}$ gemessen, was im Vergleich auf eine sehr große Anzahl kleiner Poren < 1 µm weist.

Die Dichte wurde mittels der Auftriebsmethode bestimmt.

Die Korngrößencharakterisierung erfolgte als Linienschnittanalyse an polierten und thermisch geätzten Flächen (Korngröße = 1,56 x mittlere Sehnenlänge).

Die mechanischen Eigenschaftsuntersuchungen wurden mit Ausnahme der Verschleißtests an geschliffenen Biegebruchstäben (Diamantschleifscheibe 40/50 µm/Naßschliff/Zustellung 0,01-0,02 mm) durchgeführt, was insbesondere für die Ermittlung applikationsbezogener Härtedaten sinnvoll ist, da im technischen Einsatz überwiegend geschliffene Teile verwendet werden. Die Stäbe waren zuvor durch Trennschleifen aus den gesinterten Formkörpern herausgearbeitet worden.

Zum Vergleich sind Härtewerte für polierte Oberflächen angegeben.

Die Verschleißmessungen erfolgten an polierten Oberflächen in Wasser unter Einwirkung einer geradlinig oszillierend bewegten $Al_2O_3$-Kugel (Gefügekorngröße 15 µm, Kugelradius R = 5 mm, Anpreßkraft $F_N$ = 10 N, Frequenz 20 Hz, Reibweg x = 0,2 mm). Ohne Normierung auf die Anpreßkraft, die bei den hier unter stationären Verhältnissen typischen Breiten der Verschleißspur einen Druck im Bereich von 100 - 300 MPa erzeugt, kann ein Verschleißkoeffizient als V = π b / (64 R · 2 x · n) definiert werden (b - Breite der entstehenden Verschleißspur, n - Zyklenanzahl, hier n = 100000).

Folgende Eigenschaftswerte wurden ermittelt.

| Dichte: | 3,941 g/cm$^3$ (absolut) |
|---|---|
| | 98,9 % (relativ) |
| mittlere Gefügekorngröße: | 0,65 $\mu$m |
| Härte     HV10: | 2258 $\pm$ 101 |
| HV3: | 2514 $\pm$ 124 |
| HV1: | 3055 $\pm$ 208 |
| Härte der polierten Oberfläche | |
| HV10: | 2055 $\pm$ 94 |
| Bruchfestigkeit: (3-Pkt-Biegung) | 653 $\pm$ 32 MPa. |
| Verschleißkoeffizient V: | 8 x 10$^{-7}$ mm$^3$/m. |

Vergleichstests mit einem auf 350 MPa reduzierten isostatischen Preßdruck führten zu Sinterprodukten, deren Härtewerte nicht signifikant von den vorstehenden Ergebnissen abwichen.

Eine Bewertung des Härte-Ergebnisses kann durch Gegenüberstellung mit 99 % dichten, durch heißisostatisches Pressen hergestellten geschliffenen Vergleichs-Biegebruchstäben aus $Al_2O_3$ + 35 Vol-% TiC mit einer mittleren Gefügekorngröße von 1,7 $\mu$m erfolgen. Für diese Vergleichsproben sind folgende Werte ermittelt worden:

| Härte | HV10: | 2290 $\pm$ 76 |
|---|---|---|
| | HV3: | 2386 $\pm$ 173 |
| | HV1: | 2510 $\pm$ 182. |

Somit zeigten die erfindungsgemäßen $Al_2O_3$-Sinterkörper aus gesintertem Korund, die durch druckloses Sintern an Luft hergestellt worden sind, eine Härte, welche das Niveau heißisostatisch gepreßter Komposite mit hohem Hartstoffanteil erreicht und sogar übersteigt.

Härte und Biegebruchfestigkeit der Sinterkorund-Vergleichsmuster mit ihrer o.a. höheren Defektdichte entsprachen dagegen mit nur HV10 = 1762 $\pm$ 53 bzw. $\sigma_{bB}$ = 400 $\pm$ 60 MPa dem für Gefüge gemäß dem Standes der Technik zu Erwartenden.

Zur Bewertung der Verschleißangabe kann mit der Forderung des internationalen Standards ISO-6474 an $Al_2O_3$-Implantatwerkstoffe verglichen werden. In einem ebenfalls oszillierenden Verschleißtest (anderer Anordnung bei nur 20 MPa Anpreßdruck) wird dort ein zeitbezogener Verschleiß kleiner 0,01 mm$^3$/h gefordert, was bezogen auf den Verschleißweg der Prüfanordnung einer Grenze von 2 x 10$^{-4}$ mm$^3$/m entspricht. Dagegen ist der im hier untersuchten Beispiel gefundene Verschleiß trotz eines um den Faktor 10 höheren Anpreßdruckes um drei Größenordnungen niedriger.

Beispiel 2

In 80 ml destilliertem Wasser, das mit 1n $HNO_3$ auf pH = 5 eingestellt worden war, wurden unter Rühren 200 g einer mittels Zentrifugalklassierung aus der $\alpha$-Tonerde Taimicron TM-DAR gewonnenen Kornfraktion ($d_{16}$ = 0,09 $\mu$m, $d_{50}$ = 0,12 $\mu$m, $d_{84}$ = 0,16 $\mu$m) eingebracht, wobei der pH-Wert laufend auf 5 nachgestellt wurde.

Die Suspension wurde danach noch 1 h mittels Rührwerk und gleichzeitiger Ultraschallbehandlung intensiv dispergiert, über ein 30 $\mu$m-Sieb abgesiebt und in einer Druckfiltrationszelle über einem 0,1 $\mu$m-Membranfilter bei 3,5 MPa zu Scheiben von 60 mm Durchmesser und 6 mm Dicke verpreßt.

Die Formkörper wurden über 3 Tage schrittweise zwischen 20 und 80°C auf eine Restfeuchte von 2 bis 3 % getrocknet, danach in elastischen Hüllen bei 700 MPa kaltisostatisch nachverdichtet und nochmals 5 h bei 80 °C getrocknet, was zu einer relativen Dichte von 60,2 % der theoretischen Dichte führte.

Die Formkörper wurden dann bei 800 °C in Luft vorgebrannt (Aufheizrate 0,3 K/min, 1 h Haltezeit bei 800 °C) und bei 1350 °C drucklos gesintert (Aufheizrate 2 K/min, 2 h Haltezeit bei Sintertemperatur, Ofenabkühlung).

Keramographische Untersuchungen zeigten nur vereinzelte kleine Poren der Größe 0,3 - 5 $\mu$m mit einer Häufigkeit

von 2 x 10$^{+9}$ m$^{-2}$, die dimensionslose Defektdichte betrug 4 x 10$^{-3}$. Bei Begrenzung der Auswertung auf Poren $\geq 0,8$ $\mu$m (= doppelte mittlere Korngröße) wurde hier eine im Rahmen der Meßgenauigkeit identische Häufigkeit von 3 x 10$^{+9}$ m$^{-2}$ gefunden.

Die Probenpräparation und -charakterisierung erfolgten wie in Beispiel 1.

Folgende Eigenschaftswerte wurden ermittelt:

| Dichte: | 3,940 g/cm$^3$ (absolut) |
| --- | --- |
| | 98,8 % (relativ) |
| mittlere Gefügekorngröße: | 0,40 $\mu$m |
| Härte HV10: | 2286 $\pm$ 74 |
| Verschleißkoeffizient V: | 5 x 10$^{-7}$ mm$^3$/m |

### Beispiel 3

Zu einem Gemisch aus 80 ml destilliertem Wasser, 3,7 ml Dispergierhilfsmittel Dolapix CE64 (Carbonsäurezubereitung; Fa. Zschimmer und Schwarz GmbH & Co. Chemische Fabriken, Lahnstein, Deutschland) und 18,5 ml Mg(NO$_3$)$_2$-Lösung (enthaltend 1,18 g Mg(NO$_3$)$_2$x6H$_2$O) wurden unter Rühren 370 g $\alpha$-Al$_2$O$_3$-Pulver der Sorte Taimicron TM-DAR zugemischt.

Die Suspension wurde danach noch 1 h mittels Rührwerk und gleichzeitiger Ultraschallbehandlung intensiv dispergiert, über ein 30 $\mu$m-Sieb abgesiebt und in einer Druckfiltrationszelle über einem 0,1 $\mu$m-Membranfilter bei 3,5 MPa zu Scheiben von 60 mm Durchmesser und 6 mm Dicke verpreßt.

Die Formkörper wurden über 3 Tage schrittweise zwischen 20 und 110 °C getrocknet, was zu einer relativen Dichte von 61,5 % führte. Die Formkörper wurden dann bei 800 °C in Luft vorgebrannt (Aufheizrate 0,3 K/min, 1 h Haltezeit bei 800 °C) und bei 1275 °C drucklos an Luft gesintert (Aufheizrate 2 K/min, 2 h Haltzeit bei Sintertemperatur, Ofenabkühlung).

Die keramographische Analyse des Sinterproduktes zeigte einzelne Poren $\geq 0,3$ $\mu$m mit einer Häufigkeit von 2 x 10$^{+9}$ m$^{-2}$ (dimensionslose Defektdichte ist 0,6 x 10$^{-3}$) und zusätzlich nestartige Porenansammlungen mit einer Häufigkeit von 7 x 10$^{+8}$ m$^{-2}$; die typische Größe dieser porösen Gebiete betrug 3 bis 50 $\mu$m (in weiter optimierten, hier nicht beschriebenen Verfahren dieser Art konnten diese nestartigen porösen Gebiete vermieden werden). Bei Begrenzung der Auswertung einzelner Poren auf Größen $\geq 1,5$ $\mu$m (= doppelte mittlere Korngröße) wurde hier eine wie schon im Beispiel 1 deutlich verringerte Häufigkeit von 0,2 x 10$^{+9}$ m$^{-2}$ gefunden.

Die Probenpräparation und -charakterisierung erfolgten wie im Beispiel 1.

Es wurden folgende Eigenschaftswerte ermittelt:

| Dichte: | 3,947 g/cm$^3$ (absolut) |
| --- | --- |
| | 99,0 % (relativ) |
| mittlere Gefügekorngröße: | 0,76 $\mu$m |
| Härte HV10: | 2362 $\pm$ 85 |
| Härte der polierten Oberfläche: | |
| Härte HV10: | 2105 $\pm$ 57 |

### Beispiel 4

19,2 g Acrylamid und 0,8 g N,N'Methylen-bis-acrylamid wurden in 75 ml destilliertem Wasser zusammen mit 250 g $\alpha$-Al$_2$O$_3$-Pulver der Sorte Taimicron TM-DAR eingebracht und 2 h mittels Rührwerk und gleichzeitiger Ultraschallbehandlung intensiv dispergiert, wobei der pH-Wert mit verdünnter Salpetersäure auf 4 eingestellt wird. Nach Zusatz von 0,06 g (NH$_4$)$_2$S$_2$O$_8$, gelöst in 10 ml H$_2$O, wurde der Schlicker in einer Glasschale zu einer 8 mm dicken Schicht ausgegossen und in einem Vakuumtrockenschrank bei 200 mbar zunächst bei Raumtemperatur entgast. Danach erfolgte unter Stickstoffatmosphäre die Polymerisation des Acrylamids durch Temperaturerhöhung auf 60°C. Nach einstündiger Haltezeit und langsamer Abkühlung auf Raumtemperatur wurde der scheibenförmige Körper herausgenommen und an Luft im Klimaschrank über zwei Tage bei gleichmäßig von 25 auf 90°C ansteigender Temperatur und gleichzeitig von 60 auf 15 % reduzierter relativer Feuchte getrocknet.

Die Formkörper wurden bei 800 °C in Luft vorgebrannt (Aufheizrate 0,3 K/min, 1 h Haltezeit bei 800 °C), was zu einer Gründichte von 58,4 % der theoretischen Dichte führte. Die Formkörper wurden anschließend und bei 1300 °C drucklos gesintert (Aufheizrate 2 K/min, 2 h Haltezeit bei Sintertemperatur, Ofenabkühlung).

Die Probenpräparation und -charakterisierung erfolgten wie in Beispiel 1.

Keramographische Untersuchungen im Rasterelektronenmikroskop zeigten vereinzelte kleine Poren mit typischen Größen von weniger als 3 $\mu$m, die obere Grenze für die Größe der Poren liegt im Bereich von 10 $\mu$m. Insgesamt beträgt die Häufigkeit der beobachteten Inhomogenitäten nur $2,2 \times 10^{+9}$ m$^{-2}$, unter Beachtung der Defektgrößen wurde eine dimensionslose Defektdichte von $3 \times 10^{-3}$ bestimmt.

Folgende Eigenschaftswerte wurden ermittelt:

| Dichte: | 3,968 g/cm$^3$ (absolut) |
|---|---|
|  | 99,5 % (relativ) |
| mittlere Gefügekorngröße: | 1,15 $\mu$m |
| Härte HV10: | 2143 $\pm$ 51 |
| Bruchfestigkeit: (3-Pkt-Biegung) | 810 $\pm$ 43 MPa. |

Beispiel 5

Beispiel 4 wurde mit dem Unterschied wiederholt, daß das Zwischenprodukt nach der Polymerisation bei Raumtemperatur an Luft getrocknet und einer anschließenden kaltisostatischen Nachverdichtung bei einem Druck von 700 MPa unterzogen wurde. Obwohl keine signifikante Änderung der Gründichte nachweisbar war, wurden im Vergleich mit Beispiel 4 folgende Änderungen erzielt:

- Die Häufigkeit beobachteter Inhomogenitäten ist mit $0,2 \times 10^{+9}$ m$^{-2}$ um den Faktor zehn niedriger als im Beispiel 4, während die die Defektgrößen berücksichtigende dimensionslose Defektdichte sich mit $4 \times 10^{-3}$ nicht signifikant von der des Beispiels 4 unterschied.

- eine Sinterdichte von 3,961 g/cm$^3$ entsprechend 99.3 % wurde bereits mit einer Sinterbedingung von nur 1275 °C / 2 h erreicht,

- die mittlere Korngröße des Sintergefüges betrug 0,97 $\mu$m,

- verbesserte Zuverlässigkeit in Form einer verringerten Standardabweichung der Biegebruchfestigkeit: 822 $\pm$ 19 MPa;

die Härte unterschied sich mit HV10 = 2112 $\pm$ 39 nicht signifikant von der des Beispieles 4.

Beispiel 6

Beispiel 6 entspricht weitgehend dem Verfahren von Beispiel 2, durchgeführt jedoch mit einem etwas gröberen Pulverrohstoff. In 80 ml destilliertem Wasser, das mit 1n HNO$_3$ auf pH = 4 eingestellt worden war, wurden unter Rühren 372 g einer $\alpha$-Tonerde mit einer nach BET bestimmten spezifischen Oberfläche von 10,5 m$^2$/g eingebracht, deren Korngrößenspektrum durch d$_{16}$= 0,2 $\mu$m, d$_{50}$= 0,3 $\mu$m, d$_{84}$= 0,6 $\mu$m bestimmt war.

Die Suspension wurde danach noch 1 h mittels Rührwerk und gleichzeitiger Ultraschallbehandlung intensiv dispergiert, über ein 20 $\mu$m-Sieb abgesiebt und in einer Druckfiltrationszelle über einem 0,2 $\mu$m-Membranfilter bei 1 MPa zu Scheiben von 125 mm Durchmesser und 6 mm Dicke verpreßt.

Die Formkörper wurden über 2 Tage schrittweise zwischen 20 und 90 °C auf eine Restfeuchte von 1 - 2 % getrocknet, danach in elastischen Hüllen bei 700 MPa kaltisostatisch nachverdichtet und nochmals 5 h bei 90 °C getrocknet, was zu einer relativen Dichte von 64 % der theoretischen Dichte führte.

Die Formkörper wurden dann bei 800 °C in Luft vorgebrannt (Aufheizrate 0,3 K/min, 1 h Haltezeit bei 800 °C) und bei 1450 °C drucklos gesintert (Aufheizrate 2 K/min, 2 h Haltezeit bei Sintertemperatur, Ofenabkühlung). Die anschließend ermittelte Defektdichte betrug 4 x $10^{-3}$.

Die Probenpräparation und -charakterisierung erfolgten wie in Beispiel 1.

Folgende Eigenschaftswerte wurden ermittelt:

| Dichte: | 3,960 g/cm$^3$ (absolut) |
|---|---|
| | 99,3 % (relativ) |
| mittlere Gefügekorngröße: | 1,7 μm |
| Härte HV10: | 1790 ± 48 |
| Bruchfestigkeit: (3-Pkt-Biegung) | 807 ± 67MPa. |

Beispiel 7

Das ungesinterte Vorprodukt aus Beispiel 1 wurde nach dem Vorbrennen in einem Backenbrecher gebrochen und klassiert. Die Fraktion im Bereich 0,2 - 2 mm wurde bei 1465 °C für 2 h an Luft gesintert. Das Sinterprodukt mit einer Dichte von 3,948 g/cm$^3$ entsprechend 99,0 % theoretischer Dichte wurde auf den Kornbereich 0,4 - 0,7 mm verengt und mittels eines Binders auf eine flexible Schleifscheibe aufgebracht und bzgl. seiner Schleifleistung in folgendem Test mit zwei Elektrokorundsorten verglichen:

Werkstück: Geschweißtes Stahl-Rohr aus zunderfreiem, kaltgewalztem Blech (St W 22 DIN 1543), Durchmesser 160 mm, Wandstärke 1 mm.

Schleifbedingungen: Scheibendrehzahl 6000 Umdrehungen/min, Testrohr-Drehzahl 16,3 Umdrehungen/min entsprechend einer Umfangsgeschwindigkeit von 10 m/min, Anpreßkraft 30 N.

Prüfgröße: Stahlabschliff (gemessen in Gramm) nach 1 bis 4 min.

Eine hohe Schleifleistung wird durch hohen Werkstückabtrag gekennzeichnet. Nach jeweils 1, 2, 3 und 4 min wurde folgender Gesamt-Abtrag gemessen:

Normalkorund: 41 g (1 min) - 64 g (2 min) - 83g (3 min) - 99 g (4 min).

Eutektischer Zirkonkorund: 49 g (1 min) - 76 g (2 min) - 99g (3 min) - 119 g (4 min).

Sinterkorund, erfindungsgemäß: 79 g (1 min) - 142 g (2 min) - 188g (3 min) - 226 g (4 min).

Die Ergebnisse bedeuten im zeitlichen Mittel eine Leistungssteigerung des erfindungsgemäßen Produktes auf 217 % gegenüber dem Normalkorund bzw. auf 182 % gegenüber dem Zirkonkorundmaterial.

**Patentansprüche**

1.   $Al_2O_3$-Sintermaterial mit einem Gehalt von 95 bis 100 Vol-% $Al_2O_3$, einer relativen Sinterdichte von ρ ≥ 98,5 %, und einem Gefüge mit einer mittleren Korngröße von ≤ 1,5 μm, dadurch gekennzeichnet, daß es eine Vickers-Kleinlast-Härte ≥ 2000 bei einer Prüflast von 10 bis 100 N (HV1 bis HV10), und Inhomogenitäten einer Häufigkeit von < 50 x $10^{+9}$ m$^{-2}$ aufweist, die einer oder mehrerer der folgenden Kategorien angehören:

   a) Risse und/oder poröse Gebiete entlang der Grenzen von Pulveraggregaten/Pulveragglomeraten,

   b) nestartige Gefügebereiche aufgelockerter, mit Poren durchsetzter Gefügestruktur,

   c) Poren mit einem die doppelte Gefügekorngröße übersteigenden Durchmesser.

2.   $Al_2O_3$-Sintermaterial nach Anspruch 1, dadurch gekennzeichnet, daß die Inhomogenitäten zusätzlich der folgenden Kategorie angehören können:

    d) Körner > 10 $\mu$m und/oder mehr als 10 $\mu$m große Agglomerate einzelner Körner, deren Durchmesser die fünffache mittlere Korngröße übersteigt.

**3.** $Al_2O_3$-Sintermaterial nach Anspruch 1 oder 2, dadurch gekennzeichnet, daß die Inhomogentäten der Kategorie c) zusätzlich alle Poren mit einem Durchmesser $\geq$ 0,3 $\mu$m umfassen.

**4.** $Al_2O_3$-Sintermaterial nach einem der Ansprüche 1 bis 3, dadurch gekennzeichnet, daß die Häufigkeit der Inhomogenitäten < 20 x $10^{+9}$ $m^{-2}$ ist.

**5.** $Al_2O_3$-Sintermaterial nach einem der Ansprüche 1 bis 4, dadurch gekennzeichnet, daß es ein Gefüge mit einer überwiegend regellosen Orientierung der Kristallite aufweist.

**6.** $Al_2O_3$-Sintermaterial, gekennzeichnet durch einen Gehalt von 95 bis 100 Vol-% $Al_2O_3$, eine Vickers-Kleinlast-Härte $\geq$ 1750 bei einer Prüflast von 10 N bis 100 N (HV1 bis HV10) und eine Biegebruchfestigkeit $\geq$ 800 MPa, wobei das Sintermaterial eine relative Sinterdichte von $\rho \geq$ 98,5 %, ein Gefüge mit einer mittleren Korngröße von $\leq$ 2 $\mu$m und eine dimensionslose Defektdichte von < 30 x $10^{-3}$ aufweist, wobei die Defekte einer oder mehrerer der folgenden Kategorien angehören:

    a) Risse und/oder poröse Gebiete entlang der Grenzen von Pulveraggregaten/Pulveragglomeraten,

    b) nestartige Gefügebereiche aufgelockerter, mit Poren durchsetzter Gefügestruktur,

    c) Poren mit einem die doppelte mittlere Gefügekorngröße übersteigenden Durchmesser,

    d) Körner > 10 $\mu$m und/oder mehr als 10 $\mu$m große Agglomerate einzelner Körner mit einem die fünffache mittlere Korngröße übersteigendem Durchmesser.

**7.** $Al_2O_3$-Sintermaterial nach Anspruch 6, dadurch gekennzeichnet, daß es ein Gefüge mit einer überwiegend regellosen Orientierung der Kristallite aufweist.

**8.** Verfahren zur Herstellung eines $Al_2O_3$-Sintermaterials nach einem der Ansprüche 1 bis 7, gekennzeichnet durch folgende Verfahrensschritte:

    a) Überführen eines $\alpha$-$Al_2O_3$-Pulvers mit einer mittleren Korngröße $d_{50} \leq$ 0,30 $\mu$m und einer chemischen Reinheit von $\geq$ 99,9% $Al_2O_3$ in einer Flüssigkeit in eine stabile Suspension dispergierter Partikel mittels gleichzeitiger und/oder sequentieller Anwendung von mindestens zwei unterschiedlichen Dispergierverfahren,

    b) Herstellung eines ungesinterten Vorprodukts mit einer relativen Dichte von $\rho \geq$ 55 % mittels Formgebung,

    c) Wärmebehandlung und Sinterung des Vorprodukts.

**9.** Verfahren nach Anspruch 8, dadurch gekennzeichnet, daß das in Schritt a) eingesetzte $\alpha$-$Al_2O_3$-Pulver eine durch die Parameter $d_{16}$ > 0,065 $\mu$m, $d_{50} \leq$ 0,30 $\mu$m, $d_{84}$ < 0,45 $\mu$m bestimmte Korngrößenverteilung aufweist.

**10.** Verfahren nach Anspruch 8 oder 9, dadurch gekennzeichnet, daß das in Schritt a) eingesetzte $\alpha$-$Al_2O_3$-Pulver eine nach der BET-Methode bestimmte spezifische Oberfläche von 10 - 17 $m^2$/g aufweist.

**11.** Verfahren zur Herstellung eines $\alpha$-$Al_2O_3$-Sintermaterials nach Anspruch 6 oder 7, gekennzeichnet durch folgende Verfahrensschritte:

    a) Überführen eines $\alpha$-$Al_2O_3$-Pulvers mit einer durch die Parameter $d_{16}$ > 0,065 $\mu$m, 0,2 $\mu$m $\leq d_{50} \leq$ 0,4 $\mu$m, 0,45 $\mu$m $\geq d_{84} \leq$ 0,8 $\mu$m bestimmten Korngrößenverteilung und einer chemischen Reinheit von $\geq$ 99,9% $Al_2O_3$ in einer Flüssigkeit in eine stabile Suspension dispergierter Partikel mittels gleichzeitiger und/oder sequentieller Anwendung von mindestens zwei unterschiedlichen Dispergierverfahren,

    b) Herstellung eines ungesinterten Vorprodukts mit einer relativen Dichte von $\rho \geq$ 55 % mittels Formgebung,

EP 0 756 586 B1

c) Wärmebehandlung und Sinterung des Vorprodukts.

**12.** Verfahren nach Anspruch 11, dadurch gekennzeichnet, daß das in Schritt a) eingesetzte $\alpha$-$Al_2O_3$-Pulver eine nach der BET-Methode bestimmte spezifische Oberfläche von 8 - 17 $m^2$/g aufweist.

**13.** Verfahren nach einem der Ansprüche 8 bis 12, dadurch gekennzeichnet, daß die in Schritt a) angewendeten Dispergierverfahren aus folgender Gruppe ausgewählt werden

- mechanische Rühreinrichtung,
- Beaufschlagung mit Ultraschall,
- Anwendung einer Kugelmühle.

**14.** Verfahren nach einem der Ansprüche 8 bis 13, dadurch gekennzeichnet, daß der pH-Wert der in Schritt a) hergestellten Suspension zwischen 3 und 7, vorzugsweise zwischen 4 und 6, weiter vorzugsweise bei etwa 5 liegt.

**15.** Verfahren nach einem der Ansprüche 8 bis 14, dadurch gekennzeichnet, daß zur Dispersion des $\alpha$-$Al_2O_3$-Pulvers als Flüssigkeit destilliertes oder deionisiertes Wasser unter Zusatz von Dispergierhilfsmitteln wie Mineralsäuren, Carbonsäuren, Polycarbonsäuren verwendet wird.

**16.** Verfahren nach einem der Ansprüche 8 bis 15, dadurch gekennzeichnet, daß der Dispersion chemische Substanzen zugesetzt werden, die ein oder mehrere Elemente aus der Gruppe Li, Mg, Ba, Sr, Zn, Fe, Co, Ni, Cr, Zr, Ti, Si, Y, Ce, La oder Y bis zu einer Gesamtkonzentration von 0,5 Ma.-% (bezogen auf das $Al_2O_3$) enthalten.

**17.** Verfahren nach einem der Ansprüche 8 bis 16, dadurch gekennzeichnet, daß die Formgebung mittels Druckfiltration in einem Druckbereich von 0,3 - 20 MPa, vorzugsweise 1,5 - 5,5 MPa, erfolgt.

**18.** Verfahren nach Anspruch 17, dadurch gekennzeichnet, daß der der Formgebung unterworfene Schlicker einen Feststoffgehalt von mindestens 60 Ma.-% aufweist.

**19.** Verfahren nach Anspruch 17 oder 18, dadurch gekennzeichnet, daß der druckfiltrierte Grünkörper bei Raumtemperatur an Luft bis auf eine Restfeuchte von 0,5 - 3 % getrocknet und einer anschließenden kaltisostatischen Nachverdichtung bei einem Druck von $\geq$ 200 MPa unterzogen wird.

**20.** Verfahren nach einem der Ansprüche 8 bis 16, dadurch gekennzeichnet, daß ein Preßhilfsmittel zugesetzt, der Schlicker getrocknet, und in ein Granulat überführt wird, und die Formgebung mittels kaltisostatischen Pressens bei einem Druck von $\geq$ 200 MPa erfolgt.

**21.** Verfahren nach Anspruch 20, dadurch gekennzeichnet, daß der getrocknete Versatz vor dem kaltisostatischen Pressen einer Vorverdichtung in einer uniaxial belasteten Preßform bei 20 - 200 MPa, vorzugsweise 30 - 50 MPa, unterzogen wird.

**22.** Verfahren nach einem der Ansprüche 8 bis 16, dadurch gekennzeichnet, daß der Dispersion bei einem Feststoffgehalt des $\alpha$-$Al_2O_3$-Pulvers von > 60 Masse-% ein polymerisierbares Monomer und ein Polymerisations-Initiator zugesetzt werden, der so gebildete Schlicker anschließend in eine Form gegossen, bei einem reduzierten Druck von 100 - 300 mbar entgast und schließlich die Formstabilität in einer inerten Atmosphäre durch Polymerisation infolge von Temperaturerhöhung auf 55 - 80 °C oder Zugabe eines Katalysators erreicht wird.

**23.** Verfahren nach Anspruch 22, dadurch gekennzeichnet, daß das Entgasen des Schlickers durch gleichzeitige Einwirkung von Ultraschall unterstützt wird.

**24.** Verfahren nach Anspruch 22 oder 23, dadurch gekennzeichnet, daß der hergestellte Grünkörper bei Raumtemperatur an Luft bis auf eine Restfeuchte von 0,5 - 3 % getrocknet und einer anschließenden kaltisostatischen Nachverdichtung bei einem Druck von $\geq$ 200 MPa unterzogen wird.

**25.** Verfahren zur Herstellung von Sinterformkörpern, dadurch gekennzeichnet, daß die gemäß einem der Ansprüche 22 bis 24 hergestellten grünen Formkörper vor dem Sintern einer mechanischen Bearbeitung durch Bohren, Drehen, Fräsen oder Hobeln unterzogen werden.

**26.** Verfahren nach einem der Ansprüche 8 bis 25, dadurch gekennzeichnet, daß als erster Schritt der Wärmebehandlung eine Trocknung nach folgendem Regime erfolgt: die Aufheizrate zwischen Raumtemperatur und 90 °C beträgt 1-2 K/h bei gleichzeitiger Absenkung der Umgebungsfeuchte von 60-80 % auf 15-20 %, anschließend Haltezeit von 4-6 h bei 90 °C gefolgt von einer Abkühlung auf Raumtemperatur unter Beibehaltung der niedrigen Umgebungsfeuchte.

27. Verfahren nach einem der Ansprüche 8 - 26, dadurch gekennzeichnet, daß nach dem Trocknen die Wärmebehandlung bis 800 °C mit einer Aufheizrate ≤ 0,3 K/min durchgeführt wird und die Sinterung im Temperaturbereich von 1250 - 1450 °C erfolgt.

28. Verfahren nach einem der Ansprüche 8 bis 25, dadurch gekennzeichnet, daß die Wärmebehandlung zwischen Raumtemperatur und mindestens 70 °C mit einer Aufheizrate von ≤ 2 K/h, anschließend im Bereich bis 800 °C mit einer Aufheizrate ≤ 0,5 K/min durchgeführt wird und die Sinterung im Temperaturbereich von 1200 - 1500 °C erfolgt.

29. Verfahren nach einem der Ansprüche 8 bis 25, dadurch gekennzeichnet, daß eine Wärmebehandlung zwischen Raumtemperatur und mindestens 70 °C mit einer Aufheizrate von ≤ 2 K/h, anschließend im Bereich bis zu einer Temperatur von 400 - 1000 °C mit einer Aufheizrate ≤ 0,5 K/min durchgeführt wird, die so hergestellten Zwischenprodukte nach Abkühlung auf Raumtemperatur zerkleinert, durch Klassierung in vorzugebende Korngrößenklassen fraktioniert und schließlich im Temperaturbereich von 1200 - 1500 °C gesintert werden.

30. Makroskopischer Formkörper definierter geometrischer Abmessungen aus einem $\alpha$-$Al_2O_3$-Sintermaterial nach einem der Ansprüche 1 bis 7.

31. Verwendung von Formkörpern nach Anspruch 30 für einen oder mehrere der folgenden Zwecke:

- Implantate,
- Werkzeuge oder Werkzeugteile,
- verschleißresistente Bauteile in Maschinen, Armaturen, Pumpen, Lagern,
- Fadenführer, Leit- und Umlenkelemente,
- Substrate für mikroelektronische Anwendungen,
- Träger oder Medium für einen Datenspeicher wie bspw. eine Diskette.

32. Gesinterte Körnung aus einem $\alpha$-$Al_2O_3$-Sintermaterial nach einem der Ansprüche 1 bis 7.

33. Verwendung gesinterter Körnungen nach Anspruch 32 als Schleif- oder Poliermittel.

34. Verwendung gesinterter Körnungen nach Anspruch 32 auf Produkten mit flexiblen Unterlagen.

35. Verwendung gesinterter Körnungen nach Anspruch 32 gebunden in Schleifkörpern.

**Claims**

1. Sintered $Al_2O_3$ material having a content of from 95 to 100% by volume of $Al_2O_3$, a relative sintered density of $\rho \geq$ 98.5% and a microstructure having a mean grain size of $\leq 1.5$ $\mu m$, characterized in that it has a Vickers low-load hardness of $\geq 2000$ at a test load of from 10 to 100 N (HV1 to HV10) and a frequency of $< 50 \times 10^{+9}$ $m^{-2}$ of inhomogeneities which belong to one or more of the following categories:

   a) cracks and/or porous regions along the boundaries of powder aggregates/powder agglomerates,
   b) nest-like regions in the microstructure having a loosened structure with many pores,
   c) pores having a diameter exceeding twice the grain size.

2. Sintered $Al_2O_3$ material according to Claim 1, characterized in that the inhomogeneities can additionally belong to the following category:

   d) grains of $> 10$ $\mu m$ and/or agglomerates of individual grains having an agglomerate size of greater than 10 $\mu m$ and a diameter which exceeds five times the mean grain size.

3. Sintered $Al_2O_3$ material according to Claim 1 or 2, characterized in that the inhomogeneities of the category c) additionally include all pores having a diameter of $\geq 0.3$ $\mu m$.

4. Sintered $Al_2O_3$ material according to any of Claims 1 to 3, characterized in that the frequency of the inhomogeneities is $< 20 \times 10^{+9}$ m$^{-2}$.

5. Sintered $Al_2O_3$ material according to any of Claims 1 to 4, characterized in that it has a microstructure having a predominantly arbitrary orientation of the crystallites.

6. Sintered $Al_2O_3$ material characterized by a content of from 95 to 100% by volume of $Al_2O_3$, a Vickers low-load hardness of $\geq 1750$ at a test load of from 10 N to 100 N (HV1 to HV10) and a flexural strength of $\geq 800$ MPa, where the sintered material has a relative sintered density of $\rho \geq 98.5\%$, a microstructure having a mean grain size of $\leq 2$ $\mu$m and a dimensionless defect density of $< 30 \times 10^{-3}$, where the defects belong to one or more of the following categories:

   a) cracks and/or porous regions along the boundaries of powder aggregates/powder agglomerates,
   b) nest-like regions in the microstructure having a loosened structure with many pores,
   c) pores having a diameter exceeding twice the mean grain size,
   d) grains of > 10 $\mu$m and/or agglomerates of individual grains having an agglomerate size of greater than 10 $\mu$m and a diameter which exceeds five times the mean grain size.

7. Sintered $Al_2O_3$ material according to Claim 6, characterized in that it has a microstructure having a predominantly arbitrary orientation of the crystallites.

8. Process for producing a sintered $Al_2O_3$ material according to any of Claims 1 to 7, characterized by the following process steps:

   a) conversion of an $\alpha$-$Al_2O_3$ powder having a mean particle size $d_{50}$ of $\leq 0.30$ $\mu$m and a chemical purity of $\geq$ 99.9% of $Al_2O_3$ in a liquid into a stable suspension of dispersed particles by means of simultaneous and/or sequential use of at least two different dispersing methods,
   b) production of an unsintered green intermediate product having a relative density of $\rho \geq 55\%$ by means of shaping,
   c) heat treatment and sintering of the green intermediate product.

9. Process according to Claim 8, characterized in that the $\alpha$-$Al_2O_3$ powder used in step a) has a particle size distribution determined by the parameters $d_{16} > 0.065$ $\mu$m, $d_{50} \leq 0.30$ $\mu$m, $d_{84} < 0.45$ $\mu$m.

10. Process according to Claim 8 or 9, characterized in that the $\alpha$-$Al_2O_3$ powder used in step a) has a specific surface area determined by the BET method of 10-17 m$^2$/g.

11. Process for producing a sintered $\alpha$-$Al_2O_3$ material according to Claim 6 or 7, characterized by the following process steps:

   a) conversion of an $\alpha$-$Al_2O_3$ powder having a particle size distribution determined by the parameters $d_{16} > 0.065$ $\mu$m, 0.2 $\mu$m $\leq d_{50} \leq 0.4$ $\mu$m, 0.45 $\mu$m $\leq d_{84} \leq 0.8$ $\mu$m and a chemical purity of $\geq$ 99.9% of $Al_2O_3$ in a liquid into a stable suspension of dispersed particles by means of simultaneous and/or sequential use of at least two different dispersing methods,
   b) production of an unsintered green intermediate product having a relative density of $\rho \geq 55\%$ by means of shaping,
   c) heat treatment and sintering of the green intermediate product.

12. Process according to Claim 11, characterized in that the $\alpha$-$Al_2O_3$ powder used in step a) has a specific surface area determined by the BET method of 8 - 17 m$^2$/g.

13. Process according to any of Claims 8 to 12, characterized in that the dispersing methods employed in step a) are selected from the following group

   - mechanical stirrer,
   - treatment with ultrasound,
   - use of a ball mill.

14. Process according to any of Claims 8 to 13, characterized in that the pH of the suspension produced in step a) is

EP 0 756 586 B1

between 3 and 7, preferably between 4 and 6, more preferably about 5.

15. Process according to any of Claims 8 to 14, characterized in that the liquid used for dispersing the $\alpha$-$Al_2O_3$ powder is distilled or deionized water with addition of dispersants such as mineral acids, carboxylic acids, polycarboxylic acids.

16. Process according to any of Claims 8 to 15, characterized in that chemical substances containing one or more elements selected from the group consisting of Li, Mg, Ba, Sr, Zn, Fe, Co, Ni, Cr, Zr, Ti, Si, Y, Ce, La or Y up to a total concentration of 0.5% by mass (based on the $Al_2O_3$) are added to the dispersion.

17. Process according to any of Claims 8 to 16, characterized in that the shaping is carried out by means of pressure filtration in a pressure range of 0.3 - 20 MPa, preferably 1.5 - 5.5 MPa.

18. Process according to Claim 17, characterized in that the slip subjected to shaping has a solids content of at least 60% by mass.

19. Process according to Claim 17 or 18, characterized in that the pressure-filtered green body is dried at room temperature in air to a residual moisture content of 0.5 - 3% and is subjected to subsequent further compaction by cold isostatic pressing at a pressure $\geq$ 200 MPa.

20. Process according to any of Claims 8 to 16, characterized in that a pressing aid is added, the slip is dried and converted into a granular material, and the shaping is carried out by means of cold isostatic pressing at a pressure of $\geq$ 200 MPa.

21. Process according to Claim 20, characterized in that the dried mixture is subjected to a precompaction in a uniaxially loaded pressing die at 20 - 200 MPa, preferably 30 - 50 MPa, prior to cold isostatic pressing.

22. Process according to any of Claims 8 to 16, characterized in that a polymerizable monomer and a polymerization initiator are added to the dispersion at a solids content of $\alpha$-$Al_2O_3$ powder of > 60% by mass, the slip thus formed is subsequently poured into a mould, degassed under a reduced pressure of 100 - 300 mbar and finally the shape stability is achieved in an inert atmosphere by polymerization as a result of increasing the temperature to 55 - 80°C or addition of a catalyst.

23. Process according to Claim 22, characterized in that the degassing of the slip is aided by the simultaneous action of ultrasound.

24. Process according to Claim 22 or 23, characterized in that the green body produced is dried at room temperature in air to a residual moisture content of 0.5 - 3% and is subjected to a subsequent further compaction by cold isostatic pressing at a pressure of $\geq$ 200 MPa.

25. Process for producing sintered shaped bodies. characterized in that the green shaped bodies produced according to any of Claims 22 to 24 are subjected prior to sintering to machining by drilling, turning, milling or planing.

26. Process according to any of Claims 8 to 25, characterized in that the first step of the heat treatment is drying according to the following regime: the heating rate between room temperature and 90°C is 1-2 K/h with simultaneous lowering of the ambient humidity from 60-80% to 15-20%, subsequent hold time of 4-6 hours at 90°C followed by cooling to room temperature while maintaining the low ambient humidity.

27. Process according to any of Claims 8 - 26, characterized in that after drying the heat treatment up to 800°C is carried out using a heating rate of $\leq$ 0.3 K/min and the sintering is carried out in the temperature range of 1250 - 1450°C.

28. Process according to any of Claims 8 to 25, characterized in that the heat treatment between room temperature and at least 70°C is carried out using a heating rate of $\leq$ 2 K/h, subsequently in the range up to 800°C using a heating rate of $\leq$ 0.5 K/min and the sintering is carried out in the temperature range of 1200 - 1500°C.

29. Process according to any of Claims 8 to 25, characterized in that a heat treatment between room temperature and at least 70°C is carried out using a heating rate of $\leq$ 2 K/h, subsequently in the range up to a temperature of 400 - 1000°C using a heating rate of $\leq$ 0.5 K/min, the intermediate products thus produced are, after cooling to room tem-

17

perature, comminuted, fractionated by classification into predetermined particle size classes and finally sintered in the temperature range of 1200 - 1500°C.

30. Macroscropic shaped bodies of defined geometric dimensions comprising a sintered $\alpha$-$Al_2O_3$ material according to any of Claims 1 to 7.

31. Use of shaped bodies according to Claim 30 for one or more of the following purposes:

- implants,
- tools or tool parts,
- wear-resistant components in machines, valves, pumps, bearings,
- thread guides, guide elements and deflector elements,
- substrates for microelectronic applications,
- carrier or medium for a data memory such as a computer disk substrate.

32. Sintered grains comprising a sintered polycrystalline $\alpha$-$Al_2O_3$ material according to any of Claims 1 to 7.

33. Use of sintered grains according to Claim 32 as grinding or polishing means.

34. Use of sintered grains according to Claim 32 on products with flexible substrates.

35. Use of sintered grains according to Claim 32 in bonded abrasive bodies.

## Revendications

1. Matériau fritté d'$Al_2O_3$, ayant une teneur en $Al_2O_3$ de 95 à 100 % en volume, une densité relative du fritté $\rho \geq 98,5$ %, et une texture ayant une granulométrie moyenne $\leq 1,5$ $\mu$m, caractérisé en ce qu'il a une dureté Vickers sous faible charge $\geq 2000$, pour une charge d'essai de 10 à 100 N (HV1 à HV10), avec des défauts d'homogénéité présentant une fréquence inférieure à $50 \times 10^{+9}$ $m^{-2}$, qui appartiennent à une ou plusieurs des catégories suivantes :

    a) des fissures et/ou des domaines poreux le long des joints des agrégats/agglomérats de poudre,
    b) des zones à texture en nids d'une structure à texture désagrégée traversée par des pores,
    c) des pores ayant un diamètre supérieur au double de la granulométrie de la texture.

2. Matériau fritté d'$Al_2O_3$ selon la revendication 1, caractérisé en ce que les défauts d'homogénéité peuvent en outre appartenir à la catégorie suivante :

    d) des grains > 10 $\mu$m et/ou des agglomérats de plus de 10 $\mu$m de grains individuels, dont le diamètre est supérieur à 5 fois la granulométrie moyenne.

3. Matériau fritté d'$Al_2O_3$ selon la revendication 1 ou 2, caractérisé en ce que les défauts d'homogénéité de la catégorie c) comportent en outre l'ensemble des pores ayant un diamètre $\geq 0,3$ $\mu$m.

4. Matériau fritté d'$Al_2O_3$ selon l'une des revendications 1 à 3, caractérisé en ce que la fréquence des défauts d'homogénéité est $< 20 \times 10^{+9}$ $m^{-2}$.

5. Matériau fritté d'$Al_2O_3$ selon l'une des revendications 1 à 4, caractérisé en ce qu'il comporte une texture ayant une orientation des cristallites essentiellement irrégulière.

6. Matériau fritté d'$Al_2O_3$, caractérisé en ce qu'il contient de 95 à 100 % en volume d'$Al_2O_3$, qu'il a une dureté Vickers sous faible charge $\geq 1750$ pour une charge d'essai de 10 N à 100 N (HV1 à HV10), avec une résistance à la rupture par flexion $\geq 800$ MPa, où le matériau fritté a une densité relative du fritté $\rho \geq 98,5$ %, une texture ayant une granulométrie moyenne $\leq 2$ $\mu$m, et une densité de défauts sans dimension $< 30 \times 10^{-3}$, où les défauts appartiennent à une ou plusieurs des catégories suivantes :

    a) des fissures et/ou des domaines poreux le long des joints des agrégats/agglomérats de poudre,
    b) des zones à texture en nids d'une structure à texture désagrégée traversée par des pores,
    c) des pores ayant un diamètre supérieur au double de la granulométrie de la texture,
    d) des grains > 10 $\mu$m et/ou des agglomérats de plus de 10 $\mu$m de grains individuels, dont le diamètre est supérieur à 5 fois la granulométrie moyenne.

**7.** Matériau fritté d'Al$_2$O$_3$ selon la revendication 6, caractérisé en ce qu'il comporte une texture ayant une orientation des cristallites essentiellement irrégulière.

**8.** Procédé de fabrication d'un matériau fritté d'Al$_2$O$_3$ selon l'une des revendications 1 à 7, caractérisé par les étapes suivantes :

a) conversion d'une poudre d'$\alpha$-Al$_2$O$_3$ ayant une granulométrie moyenne d$_{50}$ ≤ 0,30 μm et une pureté chimique ≥ 99,9 % d'Al$_2$O$_3$ dans un liquide, en une suspension stable de particules en dispersion, par utilisation simultanée et/ou successive d'au moins deux opérations de dispersion différentes,
b) fabrication d'une ébauche non frittée, ayant une densité relative ρ ≥ 55 %, par une opération de formage,
c) traitement thermique et frittage de l'ébauche.

**9.** Procédé selon la revendication 8, caractérisé en ce que la poudre d'$\alpha$-Al$_2$O$_3$ utilisée dans l'étape a) présente une répartition granulométrique définie par les paramètres d$_{16}$ > 0,065 μm, d$_{50}$ ≤ 0,30 μm, d$_{84}$ < 0,45 μm.

**10.** Procédé selon la revendication 8 ou 9, caractérisé en ce que la poudre d'$\alpha$-Al$_2$O$_3$ utilisée dans l'étape a) comporte une aire spécifique, déterminée par la méthode BET, de 10 à 17 m$^2$/g.

**11.** Procédé de fabrication d'un matériau fritté d'$\alpha$-Al$_2$O$_3$ selon la revendication 6 ou 7, caractérisé par les étapes suivantes

a) Conversion d'une poudre d'$\alpha$-Al$_2$O$_3$, ayant une répartition granulométrique définie par les paramètres d$_{16}$ > 0,065 μm, 0,2 μm ≤ d$_{50}$ ≤ 0,4 μm, 0,45 μm ≤ d$_{84}$ ≤ 0,8 μm, et une pureté chimique ≥ 99,9 % d'Al$_2$O$_3$ dans un liquide, en une suspension stable de particules en dispersion, par utilisation simultanée et/ou successive d'au moins deux procédés différents de dispersion,
b) fabrication d'une ébauche non frittée, ayant une densité relative ρ ≥ 55 %, par une opération de formage,
c) traitement thermique et frittage de l'ébauche.

**12.** Procédé selon la revendication 11, caractérisé en ce que la poudre d'$\alpha$-Al$_2$O$_3$ utilisée dans l'étape a) a une aire spécifique, déterminée par la méthode BET, de 8 à 17 m$^2$/g.

**13.** Procédé selon l'une des revendications 8 à 12, caractérisé en ce que les procédés de dispersion utilisés dans l'étape a) sont choisis dans le groupe suivant :

- dispositif d'agitation mécanique,
- application d'ultrasons,
- utilisation d'un broyeur à billes.

**14.** Procédé selon l'une des revendications 8 à 13, caractérisé en ce que le pH de la suspension préparée dans l'étape a) est de 3 à 7, de préférence de 4 à 6, et plus particulièrement d'environ 5.

**15.** Procédé selon l'une des revendications 8 à 14, caractérisé en ce que, pour disperser la poudre d'$\alpha$-Al$_2$O$_3$, on utilise en tant que liquide de l'eau distillée désionisée par addition d'auxiliaires de dispersion tels que des acides minéraux, des acides carboxyliques, des acides polycarboxyliques.

**16.** Procédé selon l'une des revendications 8 à 15, caractérisé en ce qu'on ajoute à la dispersion des substances chimiques qui contiennent un ou plusieurs des éléments choisis dans le groupe comprenant Li, Mg, Ba, Sr, Zn, Fe, Co, Ni, Cr, Zr, Ti, Si, Y, Ce, La ou Y jusqu'à une concentration totale de 0,5 % en masse par rapport à l'Al$_2$O$_3$.

**17.** Procédé selon l'une des revendications 8 à 16, caractérisé en ce que le formage est réalisé par filtration sous pression sur un intervalle de pression de 0,3 à 20 MPa et de préférence de 1,5 à 5,5 MPa.

**18.** Procédé selon la revendication 17, caractérisé en ce que la barbotine soumise au formage a une teneur en extrait sec d'au moins 60 % en masse.

**19.** Procédé selon la revendication 17 ou 18, caractérisé en ce que le corps vert filtré sous pression est séché à la température ambiante à l'air jusqu'à une teneur en humidité résiduelle de 0,5 à 3 %, et est ensuite soumis à une post-compression isostatique à froid, sous une pression ≥ 200 MPa.

**20.** Procédé selon l'une des revendications 8 à 16, caractérisé en ce qu'on ajoute un adjuvant de compression, on sèche la barbotine et on la transforme en un granulat, et le formage est réalisé par compression isostatique à froid sous une pression ≥ 200 MPa.

**21.** Procédé selon la revendication 20, caractérisé en ce que la pâte séchée est, avant compression isostatique à froid, soumise à une précompression dans un moule de compression soumis à une charge uniaxiale, sous une pression de 20 à 200 et de préférence de 30 à 50 MPa.

**22.** Procédé selon l'une des revendications 8 à 16, caractérisé en ce qu'on ajoute à la dispersion, pour une teneur en extrait sec de la poudre d'$\alpha$-Al$_2$O$_3$ > 60 % en masse, un monomère polymérisable ou un amorceur de polymérisation, on verse ensuite la barbotine ainsi formée dans un moule, on la dégaze sous une pression réduite de 100 à 300 mbar, puis finalement on arrive à la stabilité de forme dans une atmosphère inerte par polymérisation en conséquence d'une élévation de la température à 55-80°C ou de l'addition d'un catalyseur.

**23.** Procédé selon la revendication 22, caractérisé en ce que, le dégazage de la barbotine est facilité par l'utilisation simultanée d'ultrasons.

**24.** Procédé selon la revendication 22 ou 23, caractérisé en ce que le corps vert ainsi fabriqué est séché à la température ambiante à l'air jusqu'à une humidité résiduelle de 0,5 à 3 %, et est soumis ensuite à une post-compression isostatique à froid sous une pression ≥ 200 MPa.

**25.** Procédé de fabrication d'un comprimé fritté, caractérisé en ce que les comprimés verts, fabriqués selon l'une des revendications 22 à 24, sont soumis avant le frittage à un usinage mécanique par perçage, tournage, fraisage ou rabotage.

**26.** Procédé selon l'une des revendications 8 à 25, caractérisé en ce qu'on procède en tant que première étape du traitement thermique à un séchage selon le schéma suivant : la vitesse de montée en température entre la température ambiante et 90°C est de 1 à 2 K/h, avec un abaissement simultané de l'humidité ambiante de 60-80 % à 15-20 %, puis un palier de 4 à 6 heures à 90°C, suivi d'un refroidissement à la température ambiante tout en maintenant la faible humidité ambiante.

**27.** Procédé selon l'une des revendications 8 à 26, caractérisé en ce que, après le séchage, on procède au traitement thermique jusqu'à 800°C à une vitesse de montée en température ≤ 0,3 K/min, le frittage étant réalisé dans l'intervalle de température de 1250 à 1450°C.

**28.** Procédé selon l'une des revendications 8 à 25, caractérisé en ce que le traitement thermique est réalisé entre la température ambiante et au moins 70°C à une vitesse de montée en température ≤ 2 K/h, puis dans la plage de température allant jusqu'à 800°C à une vitesse de montée en température ≤ 5 K/min, le frittage étant réalisé dans l'intervalle de température de 1200 à 1500°C.

**29.** Procédé selon l'une des revendications 8 à 25, caractérisé en ce qu'on procède à un traitement thermique entre la température ambiante et au moins 70°C à une vitesse de montée en température inférieure ou égale à 2 k/h, puis dans l'intervalle allant jusqu'à une température de 400 à 1000°C à une vitesse de montée en température ≤ 0,5 K/min, puis, après refroidissement à la température ambiante, on broie les produits intermédiaires ainsi fabriqués, on les fractionne par classification en des classes granulométriques à prédéfinir, et finalement on les fritte à une température comprise entre 1200 et 1500°C.

**30.** Comprimés macroscopiques ayant des dimensions géométriques définies, constitués d'un matériau fritté d'$\alpha$-Al$_2$O$_3$ selon l'une des revendications 1 à 7.

**31.** Utilisation de comprimés selon la revendication 30 pour une ou plusieurs des applications suivantes :

- Implants
- Outils ou parties d'outils
- Composants résistant à l'usure dans les machines, les robinetteries, les portes, les paliers,
- Guide-fils, éléments déflecteurs et de renvois,
- Substrats pour applications micro-électroniques,

- Supports pour mémoires de données, par exemple des disquettes.

32. Produits agglomérés frittés constitué d'un matériau fritté d'$\alpha$-Al$_2$O$_3$ selon l'une des revendications 1 à 7.

33. Utilisation de produits agglomérés frittés selon la revendication 32 en tant qu'agents abrasifs et de polissage.

34. Utilisation de produits agglomérés frittés selon la revendication 32, sur des produits comportant des supports souples.

35. Utilisation de produits agglomérés frittés selon la revendication 32, liés dans des produits abrasifs.